# EUROPÄISCHE PATENTSCHRIFT

(11) **EP 2 270 151 B1**
(45) Veröffentlichungstag und Bekanntmachung des Hinweises auf die Patenterteilung: **04.07.2012**
(21) Anmeldenummer: 10165393.9
(22) Anmeldetag: 09.06.2010
(51) Int. Cl.: C12N 15/10, C12Q 1/68

(54) **Verfahren zur Entparaffinisierung biologischer Proben**
Method for removing paraffin from biological samples
Procédé de déparaffinage d'échantillons biologiques

(30) Priorität: 01.07.2009 DE 102009031434
(43) Veröffentlichungstag der Anmeldung: 05.01.2011
(73) Patentinhaber: AXAGARIUS GmbH & Co. KG, 52355 Düren (DE)
(72) Erfinder: Kirsch, Christoph, 50529 Puhlheim (DE); Beyard, Claudia, 53909 Zülpich (DE); Meusel, Markus, 52146 Würselen (DE); Möller, Klaus, 52249 Eschweiler (DE); Radmacher, Edmund, 52349 Düren (DE)
(74) Vertreter: Albrecht, Ralf

(56) Entgegenhaltungen:
- EP-A2- 1 743 939
- WO-A1-01/46402
- WO-A2-2006/039563
- WO-A2-2006/130632
- WO-A2-2008/021419
- CHRISTIANE SCHEWE ET AL: "Inter-laboratory validation of PCR-based detection of Mycobacterium tuberculosis in formalin-fixed, paraffin-embedded tissues" VIRCHOWS ARCHIV, SPRINGER, BERLIN, DE LNKD- DOI:10.1007/S00428-005-1233-3, Bd. 447, Nr. 3, 1. September 2005 (2005-09-01), Seiten 573-585, XP019344790 ISSN: 1432-2307
- SATO Y ET AL: "COMPARISON OF THE DNA EXTRACTION METHODS FOR POLYMERASE CHAIN REACTION AMPLIFICATION FROM FORMALIN-FIXED AND PARAFFIN-EMBEDDED TISSUES" DIAGNOSTIC MOLECULAR PATHOLOGY, NEW YORK, NY, US LNKD- DOI:10.1097/00019606-200112000-00009, Bd. 10, Nr. 4, 1. Dezember 2001 (2001-12-01), Seiten 265-271, XP009010319
- BioGenex Laboratories Inc.: "Safety data sheet EZ DEWAX SOLUTION, READY TO USE" 30. Oktober 2003 (2003-10-30), XP002599848 Gefunden im Internet: URL:http://www.biogenex.com/int_msds/931-H K585-EN-1232081898.pdf?PHPSESSID=b382f56e2 aa222aec2561f35b9c753e4 [gefunden am 2010-09-08]
- LEHMANN U ET AL: "Real-time PCR analysis of DNA and RNA extracted from formalin-fixed and paraffin-embedded biopsies.", METHODS (SAN DIEGO, CALIF.) DEC 2001 LNKD- PUBMED:11846610, vol. 25, no. 4, December 2001 (2001-12), pages 409-418, ISSN: 1046-2023
- BANERJEE S K ET AL: "MICROWAVE-BASED DNA EXTRACTION FROM PARAFFIN-EMBEDDED TISSUE FOR PCR AMPLIFICATION", BIOTECHNIQUES, INFORMA HEALTHCARE, US, vol. 18, no. 5, 1 January 1995 (1995-01-01), XP009011206, ISSN: 0736-6205

## Beschreibung

Die vorliegende Erfindung betrifft ein Verfahren zur Isolierung von Biomolekülen aus einer fixierten, in Paraffin eingebetteten biologischen Probe, ein Kit, welches die zur Durchführung des erfindungsgemäßen Verfahrens benötigten Substanzen beinhaltet sowie die Verwendung des erfindungsgemäßen Kits zur Durchführung des Isolationsverfahrens.

Aus dem Stand der Technik sind Verfahren zur Isolierung von Biomolekülen aus fixierten, in Paraffin eingebetteten biologischen Proben bekannt. Unter einer fixierten Probe wird vorliegend eine biologische Probe verstanden, welche auf an sich bekannte Weise durch Behandlung, beispielsweise mit einer Formaldehydlösung, wasserfreiem Ethanol oder sauren alkoholischen Lösungen haltbar gemacht wird. Zur weiteren Verbesserung der Lagerungsstabilität werden diese Proben anschließend in Paraffin überführt. Die mit Formalin behandelten und in Paraffin eingebetteten Proben werden auch als FFPE- ("Formalin-fixiertes, Paraffineingebet-tetes") Material bezeichnet. Die mittels dieser Methoden fixierten und eingebetteten Proben können beispielsweise für histopathologische Untersuchungen eingesetzt und/oder anschließend über einen sehr langen Zeitraum aufbewahrt werden, ohne dass es zu einer nennenswerten Veränderung der in diesen Proben enthaltenen Biomoleküle kommt. Insbesondere lassen sich selbst nach längeren Zeiträumen noch die Nukleinsäuren, d.h. RNA und DNA aus diesen Proben extrahieren.

Die Gewinnung dieser Biomoleküle aus solchen fixierten, in Paraffin eingebetteten biologischen Proben ist jedoch aufwändig, da die Proben zunächst vom sie umgebenden Paraffin befreit werden müssen. Bei den Proben handelt es sich üblicherweise um dünne Schnitte, deren Paraffinanteil in der Regel den Probenanteil deutlich übersteigt. Da das Paraffin bei der weiteren Probenaufbereitung und Isolation der Biomoleküle stören kann oder diese gänzlich unterbindet, werden im Stand der Technik unterschiedliche Verfahrensweisen vorgeschlagen, mittels derer das Paraffin abgetrennt werden kann.

In der WO 2006/039563 wird vorgeschlagen, die biologische Probe nach dem Schneiden, beispielsweise mit einem Mikrotom, mit Xylol, Toluol, Isoparaffin oder Limonen vorzubehandeln, um das Paraffin aufzulösen. Diese Lösung wird anschließend zentrifugiert, das Lösungsmittel entfernt und die Probe gegebenenfalls mit Ethanol gewaschen, um möglicherweise vorhandene Lösemittelreste zu entfernen. Anschließend wird die Probe getrocknet und dann der weiteren Analyse unterzogen.

Die für die Gewinnung von sauberem Probenmaterial erforderlichen mehrfachen Waschschritte mit Ethanol sind einerseits aufwändig und bergen darüber hinaus die Gefahr, dass nach dem Abzentrifugieren beim Abziehen des Ethanolüberstandes versehentlich bereits Teile des Probenmaterials mit entfernt werden. Die gleiche Problematik ergibt sich bei der Zentrifugation und Abnahme des Lösungsmit-Zentrifugation und Abnahme des Lösungsmittels für das Paraffin. Dies ist insbesondere problematisch, wenn nur wenig Probenmaterial zur Verfügung steht, weil dann das Probenmaterial im Lösungsmittel mit dem bloßen Auge kaum erkennbar ist. Da eine saubere Probenaufarbeitung über dieses Verfahren viel Geschick und vor allem Erfahrung erfordert, ist eine zuverlässige Automatisierung kaum möglich.

In der US 2007/0026432 wird ein weiteres Verfahren zur Analyse von fixierten, in Paraffin eingebetteten biologischen Proben beschrieben. Dazu wird vorgeschlagen, das in Paraffin eingebettete Probenmaterial nach Zugabe von Detergenzien über den Schmelzpunkt des Paraffins zu erhitzen und die Probe anschließend wieder unter den Schmelzpunkt des Paraffins abkühlen zu lassen. Die Gewinnung des Probenmaterials erfolgt dann mit Hilfe einer Kanüle, die durch die erstarrte Paraffinschicht in das darunter befindliche Probenmaterial geführt wird. Nachteilig bei diesem Verfahren ist, dass beim Durchstechen der Paraffinschicht die Kanüle durch Paraffin verstopft werden kann, was eine Automatisierung praktisch unmöglich macht.

Aus EP 1 743 939 und WO 01/46402 A1 ist ebenfalls ein Verfahren zur Entparaffinisierung biologischer Proben bekannt. In dieser Schrift wird vorgeschlagen, das Paraffin mit Hilfe geeigneter Lösungsmittel wie Benzol, Toluol, Ethylbenzol, Xylol oder Mischungen hiervon aufzulösen und mittels Zentrifugation abzutrennen, wobei das bei der Zentrifugation erhaltene Pellet anschließend mehrfach mit einem geeigneten Lösungsmittel gewaschen wird, um das Paraffin möglichst vollständig zu entfernen.

Aus dem Artikel "Inter-laboratory validation of PCR-based detection of Mycobacterium tuberculosis in formalin-fixed, paraffin-embedded tissues", Christiane Schewe et al., Virchows Archiv, Springer, Berlin, Bd. 447, Nr. 3, September 2005, Seiten 573-585 ist ein Verfahren zur Durchführung einer PCR-Reaktion an DNA aus paraffineingebetteten Proben bekannt. Dabei werden die Proben zunächst mit Xylol versetzt, um das Paraffin aufzulösen, anschließend zentrifugiert, der Überstand verworfen und das erhaltene Pellet mit Ethanol gewaschen. Sowohl die Xylolextraktion als auch die Waschschritte mit Ethanol können ein- oder mehrfach durchgeführt werden.

Aus dem Artikel "Comparison of the DNA extraction methods for polymerase chain reaction amplification from formalin-fixed and paraffin-embedded tissues" von Sato et al., Diagnostic molecular pathology, New York, Band 10, Nr. 4, Dezember 2011, Seiten 265-271 ist ein hierzu analoges Verfahren zur Entfernung des Paraffins aus paraffineingebetteten Proben bekannt, bei dem ebenfalls das Paraffin zunächst mit Xylol entfernt, anschließend zentrifugiert, der Überstand verworfen, das bei der Zentrifugation erhaltene Pellet mit Ethanol zur Entfernung der Xylols ein- oder mehrfach gewaschen wird.

Aus WO 2006/130632 A2 ist ein Verfahren zur Isolierung von Biomolekülen aus einer fixierten, in Paraffin eingebetteten biologischen Probe bekannt. Bei dem hier beschriebenen Verfahren wird zunächst die zu analysierende Probe auf 50 bis 85°C für etwa eine bis sechzig Minuten in Gegenwart eines ionischen detergenzhaltigen Puffers erhitzt. Dabei bildet sich ein zweiphasiges Gemisch. Die Isolierung der Biomoleküle erfolgt anschließend aus der wässrigen Phase.

In dem Artikel "Real-Time PCR analysis of DNA and RNA extracted from formalin-fixed and paraffin-embedded biopsies" von Lehmann et al., Methods (San Diego), Dezember 2011, Band 25, Nr. 4, Dezember 2001, Seiten 409-418 ist ein Verfahren zur Isolation von RNA aus Formalin-fixierten und in Paraffin eingebetteten Proben bekannt. Dabei werden die Proben zunächst auf 55°C über Nacht in Gegenwart eines ionischen Detergenz/Protease-Gemischs erhitzt und dann mit einer wässrigen Lösung und/oder Wasser in Kontakt gebracht, wobei sich ein zweiphasiges Gemisch bildet, wobei anschließend die Isolierung der Biomoleküle aus der wässrigen Phase erfolgt. Auch hierbei wird durch das Erhitzen in Gegenwart des Detergenz-haltigen Puffers das Paraffin flüssig und die Zellen der Gewebeprobe beginnen zu lysieren.

Ein weiteres Verfahren zur Isolation von RNA aus Formalin-fixierten und in Paraffin eingebetteten Proben ist aus dem Artikel "Microwave-based DNA extraction from paraffin-embedded tissue for PCR amplification" von Banerjee et al., Biotechniques, Informa Healthcare, US, Band 18, Nr. 5, Januar 1995, bekannt. Bei diesem Verfahren wird die Probe zunächst in einer Mikrowelle (500W, 30 bis 60 Sekunden) in Gegenwart eines nichtionischen Detergenzes erhitzt. Nach Zentrifugation wird das erstarrte Paraffin entfernt und die wässrige Phase mit einem Protenase-haltigen Lyse-Puffer versetzt. Die anschließende Isolierung der Biomoleküle erfolgt aus der wässrigen Phase.

Ein weiteres Verfahren zur Isolation von RNA aus Formalin-fixierten und in Paraffin eingebetteten Proben ist aus WO 2008/021419 A2 bekannt. Bei dem hier vorgestellten Verfahren wird die Probe zunächst auf eine Temperatur von über 40°C in Gegenwart eines ionischen Detergenz/Protease-Gemischs erhitzt. Hierbei bildet sich ein zweiphasiges Gemisch, wobei die anschließende Isolierung der Biomoleküle aus der wässrigen Phase erfolgt.

Die Aufgabe der vorliegenden Erfindung besteht darin, ein Verfahren zur Isolierung von Biomolekülen aus einer fixierten, im Paraffin eingebetteten biologischen Probe zu schaffen, das eine möglichst quantitative Gewinnung der Biomoleküle aus der Probe erlaubt, Verluste, wie sie durch das Abnehmen einer organischen Phase mit dem darin gelösten Paraffin möglich sind, vermeidet und das ferner für eine automatisierte Probenaufarbeitung geeignet ist.

Diese Aufgabe wird erfindungsgemäß durch ein Verfahren zur Isolierung von Biomolekülen aus einer fixierten, in Paraffin eingebetteten biologischen Probe gelöst, das die folgenden Schritte umfasst:
Inkontaktbringen der Probe
   a) mit einem nicht mit Wasser mischbaren Lösungsmittel und Lösen des Paraffins in diesem Lösungsmittel unter Ausbildung einer flüssigen organischen Phase, und
   b) mit einer wässrigen Lösung mindestens einer Lysesubstanz und/oder mit Wasser und mindestens einer Lysesubstanz unter Ausbildung einer wässrigen Phase;
Isolierung der Biomoleküle aus der wässrigen Phase.

Der vorliegenden Erfindung liegt die Erkenntnis zugrunde, dass zur Isolierung von Biomolekülen aus einer in Paraffin eingebetteten biologischen Probe diese zur Auflösung des Paraffins mit einem nicht mit Wasser mischbaren Lösungsmittel versetzt werden kann, welches mit dem Paraffin zusammen eine organische Phase bildet, die während der weiteren Probenaufarbeitung im Probengefäß verbleiben kann. Eine Entfernung des Lösungsmittels ist daher ebenso wenig erforderlich, wie die Durchführung sukzessiver Waschschritte, wodurch sich der Verfahrensaufwand erheblich reduzieren lässt. Durch den Verzicht auf die im Stand der Technik bei Einsatz von Lösungsmitteln vorgesehenen mehrfachen Waschschritte lässt sich die Gefahr des Verlustes von Probenmaterial weitestgehend ausschließen.

Das erfindungsgemäße Verfahren stellt ferner sicher, dass die organische Phase aus Paraffin und nicht mit Wasser mischbarem Lösungsmittel bei üblichen Umgebungstemperaturen flüssig ist. Bei Probenentnahme durch die organische Phase, wie beispielsweise mittels einer durch diese hindurchgeführte Kanüle oder Pipette, kann ein Verstopfen durch Paraffin somit ausgeschlossen werden. Das erfindungsgemäße Verfahren lässt sich daher auch automatisiert unter Einsatz entsprechender Pipettierroboter durchführen. Dass das erfindungsgemäße Verfahren auch ohne Zentrifugationsschritte durchgeführt werden kann, erleichtert zudem eine Automation. Insbesondere ist die organische Phase bei einer Temperatur von ≤ 25°C, bevorzugt bei ≤ 20°C oder bei ≤ 15°C flüssig.

Mit Hilfe des erfindungsgemäßen Verfahrens lassen sich im Prinzip sämtliche Arten von Biomolekülen aus biologischen Proben isolieren. Dies sind beispielsweise Nukleinsäuren und/oder Proteine. Als Nukleinsäuren kommen RNA und DNA unterschiedlicher Kettenlänge in Frage, insbesondere mit mehr als fünfzehn Nukleotiden, wie beispielsweise einzel- und doppelsträngige bakterielle, virale, humane, tierische oder pflanzliche RNA oder DNA, insbesondere genomische DNA, mitochondriale DNA, Plasmide, mRNA, tRNA, rRNA, miRNA und andere kurze RNA-Spezies, insbesondere mit einer Kettenlänge von 15 bis 25 Nukleotiden. Alle zuvor genannten Biomoleküle können einzeln oder auch in beliebiger Kombination durch das erfindungsgemäße Verfahren isoliert werden. Die Proben liegen beispielsweise in Form dünner Schnitte der in Paraffin eingebetteten biologischen Probe vor, wie sie mittels eines Mikrotoms erhalten werden.

Unter einem nicht mit Wasser mischbaren Lösungsmittel wird ein Lösungsmittel verstanden, das bei 25°C bei einem Mischungsverhältnis von 1:1 mit Wasser zwei voneinander getrennte Phasen ausbildet. Die im Rahmen der vorliegenden Erfindung eingesetzten Lösungsmittel sollten in vorteilhafter Weise darüber hinaus gute Lösungseigenschaften in Bezug auf die üblicherweise zum Einbetten biologischer Proben verwendeten Paraffine besitzen. Dazu sollte zur Lösung von 1mg Paraffin bei 25°C eine Lösungsmittelmenge von nicht mehr als 500 µl erforderlich sein, bevorzugt nicht mehr als 300µl, besonders bevorzugt nicht mehr als 150 µl.

Unter einer Lysesubstanz wird im Sinn der vorliegenden Erfindung eine Verbindung verstanden, die in der Lage ist, Biomoleküle aus dem Material der biologischen Probe freizusetzen. Dies können ein oder mehrere Enzyme sein, beispielsweise Proteasen.

Als Lysesubstanzen können auch ein oder mehrere Chaotropsalze verwendet werden. Zu diesem Zweck kommen vor allem Guanidiniumsalze wie Guanidiniumthiocyanat oder Guanidiniumhydrochlorid, Natrium- oder Kaliumthiocyanat, Kaliumiodid, Perchlorate oder Bariumsalze in Frage.

Andere, die Lyse unterstützende Substanzen können ebenfalls im Sinn der Erfindung eingesetzt werden.

Die zuvor genannten Lysesubstanzen, d.h. z.B. Enzyme bzw. Chaotropsalze können alternativ oder in beliebigen Kombinationen eingesetzt werden. Es ist erfindungsgemäß vorgesehen, dass die Lysesubstanz bereits in Form einer wässrigen Lösung vorliegt oder durch Zugabe von Wasser und Lysesubstanz eine wässrige Lösung in-situ gebildet wird. In beiden Fällen kommt es somit zur Ausbildung einer wässrigen Phase.

Sofern im Rahmen des erfindungsgemäßen Verfahrens sowohl Enzyme als auch Chaotropsalze eingesetzt werden, ist es zweckmäßig, wenn zunächst das bzw. die Enzyme hinzugegeben werden sowie gewünschtenfalls eine geringe Menge an Chaotropsalz und anschließend inkubiert wird. In einem zweiten Schritt kann dann die Chaotropkonzentration bis auf das gewünschte Maß erhöht werden oder, wenn zunächst nur Enzyme hinzugefügt wurden, in diesem Schritt die gesamte Menge an Chaotropsalz zugesetzt werden. Dies gilt sowohl für den Fall, dass wässrige Lösungen dieser Substanzen eingesetzt werden als auch im Falle des Einsatzes von Wasser und festen Lysesubstanzen. Auf diese Weise kann eine mögliche Aktivitätsreduktion des oder der Enzyme durch eine zu hohe anfängliche Chaotropsalzkonzentration verhindert werden.

Bei biologischen Proben, welche mittels einer Formalin-Lösung fixiert wurden, sollte im Schritt b) zweckmäßigerweise eine Lysesubstanz eingesetzt werden, welche die Entfernung von durch die Formalinbehandlung entstandenen Vernetzungen unterstützt, wie beispielsweise Enzyme, insbesondere eine Protease. Bei mit Ethanol oder sauren alkoholischen Lösungen fixierten biologischen Proben kann die Zugabe eines Chaotropsalzes im Verfahrensschritt b) genügen, um die in der biologischen Probe enthaltenen Biomoleküle freizusetzen.

Im Rahmen des erfindungsgemäßen Verfahrens ist es möglich, dass der Schritt a) vor, nach oder gleichzeitig mit dem Schritt b) durchgeführt wird. Zweckmäßigerweise wird Schritt a) jedoch vor dem Schritt b) durchgeführt, da auf diese Weise das Paraffin von der biologischen Probe entfernt werden kann, bevor mit der Lyse der Probe im Schritt b) fortgefahren wird.

In einer Ausgestaltung des erfindungsgemäßen Verfahrens weist das nicht mit Wasser mischbare Lösungsmittel eine geringere Dichte als Wasser und/oder die wässrige Lösung auf. Die Dichte des nicht mit Wasser mischbaren Lösungsmittels beträgt insbesondere ≤ 0,95 g/cm³ oder ≤ 0,90 g/cm³. Bei der Zugabe eines solchen Lösungsmittels zu der in Paraffin eingebetteten biologischen Probe bildet diese als organische Phase nach anschließender Zugabe des Wassers bzw. der wässrigen Lyselösung einen Überstand. Da die biologische Probe üblicherweise eine Dichte besitzt, die entweder gleich oder größer als die von Wasser ist, befindet sich das Probenmaterial in der wässrigen Phase, wodurch das Lysieren der Probe durch bessere Einwirkung der in der wässrigen Phase enthaltenen Lysesubstanz beschleunigt wird und vollständiger abläuft. Hierzu kann die Mischung auch geschüttelt werden, falls die Probe zunächst aufgrund der Oberflächenspannung auf der Flüssigkeitsoberfläche schwimmt. Außerdem sind die biologischen Proben in der Regel hydrophil und können so leicht hydratisiert werden, was ebenfalls den Übergang aus der organischen Phase in die wässrige Phase erleichtert. Dies gilt auch in dem Fall, dass das nicht mit Wasser mischbare Lösungsmittel eine höhere Dichte als Wasser bzw. die wässrige Lösung aufweist.

Der durch die organische Phase gebildete Überstand schützt die Probe gleichzeitig vor der Einwirkung von Luftsauerstoff, der zu einer oxidativen Veränderung der zu isolierenden Biomolekülen führen kann. Da die Probengefäße üblicherweise im Bodenbereich spitz zulaufen, ist außerdem eine leichtere Entnahme der wässrigen Phase möglich.

Die Isolierung der Biomoleküle aus der wässrigen Phase kann in an sich bekannter Weise erfolgen. Hierzu werden die zu isolierenden Biomoleküle beispielsweise an einen festen Träger gebunden. Hierfür können Silicamembranen oder lose Silicapartikel, Polymer-, Glaspartikel oder Magnetbeads eingesetzt werden. Die wässrige Phase kann für diesen Schritt entweder aus dem Probengefäß entnommen und auf die feste Phase gebracht werden, wozu gegebenenfalls noch geeignete Bindebedingungen eingestellt werden. Dies wird beispielsweise durch Zugabe eines Chaotropsalzes und/oder eines oder mehrerer Alkohole mit einem bis vier Kohlenstoffatomen wie insbesondere Methanol oder Ethanol zur wässrigen Phase erreicht. Das Einstellen der Bindebedingungen kann bevorzugt in Gegenwart der organischen Phase erfolgen.

Alternativ hierzu kann dem zweiphasigen Gemisch aus organischer und wässriger Phase der feste Träger, beispielsweise in Form von Magnetbeads, zugefügt werden, wobei auch in diesem Fall die Bindebedingungen in Abhängigkeit von der Oberflächenbeschaffenheit der Beads z.B. durch Zugabe von Chaotropsalzen und/oder den oben genannten Alkoholen verbessert werden können. Die Biomoleküle binden dann in dem zweiphasigen Gemisch an den festen Träger und können anschließend abgetrennt werden.

Die Isolierung der Biomoleküle aus der wässrigen Phase ist jedoch nicht zwingend an das Vorhandensein einer festen Phase gebunden. Nukleinsäuren können beispielsweise aus der wässrigen Phase mit an sich bekannten Verfahren beispielsweise präzipiziert oder extrahiert werden.

In weiterer Ausgestaltung des erfindungsgemäßen Verfahrens ist das nicht mit Wasser mischbare Lösungsmittel bei ≤ 25°C flüssig, insbesondere bei ≤ 20°C, bevorzugt bei einer Temperatur von ≤ 15°C, besonders bevorzugt bei ≤ 10°C. Auf diese Weise kann sichergestellt werden, dass die organische Phase auch bei kühleren Umgebungstemperaturen selbst nach Lösen des Paraffins in flüssiger Form verbleibt.

In Weiterbildung des erfindungsgemäßen Verfahrens ist das nicht mit Wasser mischbare Lösungsmittel ausgewählt aus aromatischen Kohlenwasserstoffverbindungen mit 6 bis 30 Kohlenstoffatomen, aliphatischen Kohlenwasserstoffverbindungen mit 10 bis 30 Kohlenstoffatomen, insbesondere aus Alkanen oder Alkenen. Besonders geeignet sind lineare Kohlenwasserstoffverbindungen mit 9 bis 15 Kohlenstoffatomen, insbesondere mit 12 bis 15 Kolenstoffatomen oder deren Mischungen. Insbesondere sind die zuvor genannten linearen Kohlenwasserstoffverbindungen gesättigt oder einfach ungesättigt. Dies sind beispielhaft n-Nonan, n-Decan, n-Undecan, n-Dodecan, n-Tridecan und besonders bevorzugt n-Tetradecan, n-Pentadecan sowie n-Pentadecen. Aber auch technische Lösungsmittel wie Biodiesel, Diesel bzw. Heizöl, dünnflüssiges Paraffin, Weißöl, Petroleum, Kerosin oder Sinarol können eingesetzt werden. Bei Sinarol handelt es sich um ein C₁₄ - C₁₉ Kohlenwasserstoffgemisch mit einem Siedebereich von 250°C - 330°C.

Außerdem können Alkan- oder Alkensäuren mit 2 bis 20, insbesondere 6 bis 18 Kohlenstoffatomen und deren Derivate, aromatische oder aliphatische Alkohole mit 6 bis 12 Kohlenstoffatomen eingesetzt werden. Es sind ebenso Mischungen aller zuvor genannten Substanzen möglich. Beispiele für einsetzbare Alkansäuren sind Octan-, Nonan- oder Decansäure.

Die aliphatischen Kohlenwasserstoffverbindungen können linear, verzweigt, cyclisch, gesättigt oder ungesättigt sein sowie aus Mischungen von diesen bestehen. Sofern aromatische Kohlenwasserstoffverbindungen mit aliphatischen Substituenten eingesetzt werden, können diese ebenfalls linear, verzweigt, cyclisch, gesättigt oder ungesättigt sein. Beispiele für einsetzbare cyclische bzw. aromatische Kohlenwasserstoffe sind Pinen und Limonen einerseits, sowie Xylol und Cymol andererseits.

Es ist ebenso möglich, dass die aromatischen und/oder aliphatischen Kohlenwasserstoffverbindungen Heteroatome tragen, wie beispielsweise Schwefel, Stickstoff, Sauerstoff, Fluor, Chlor, Brom und/oder Iod enthaltene Gruppen.

Bevorzugte Alkan- oder Alkensäurederivate sind deren Ester, insbesondere deren Alkylester aus linearen Carbonsäuren und linearen Alkoholen mit jeweils einem bis 14 Kohlenstoffatomen, wobei der Ester insbesondere 5 bis 20 Kohlenstoffatome aufweist. Dies sind beispielsweise Essigsäure-Butylester, Propionsäure-Etylester, Propionsäure-Pentylester, Propionsäure-Octylester, Propionsäure-Decylester, Butylsäure-Decylester, Pentylsäure-Octylester, Octylsäure-Hexylester, Octylsäure-Octylester, Decylsäure-Butylester sowie Dodecylsäure-Butylester.

Des weiteren sind auch gesättigte und/oder verzweigte organische Säureester, wie beispielsweise Octylsäure-Isoamylester oder aber auch Ester mehrwertiger organischer Säuren, wie Acetyl-Tributyl-Citrat, Adipinsäurediethylester oder Malonsäure-Diethylester einsetzbar.

Zur Verbesserung einer deutlichen Phasentrennung zwischen organischer Phase und wässriger Phase ist es vorteilhaft, wenn die eingesetzten organischen Lösungsmittel einen Kow-Wert (n-Octanol-Wasser-Verteilungskoeffizient) von > 1,0, insbesondere von ≥ 2,0 aufweisen. Besonders bevorzugt sind Lösungsmittel mit einem K_{OW}-Wert von ≥ 4,0, insbesondere von ≥ 5,0 oder von ≥ 6,0. Beim Einsatz solcher Lösungsmittel wird eine deutliche Trennung zwischen organischer und wässriger Phase auch bei erhöhten Temperaturen oder bei Zugabe von Chaotropsalz oder Alkoholen wie Ethanol oder Methanol erreicht, wodurch ein Verschleppen von Biomolekülen in die organische Phase weitestgehend ausgeschlossen werden kann. Lösungsmittel mit einem K_{OW}-Wert von ≥ 5,0 sind beispielsweise n-Nonan, n-Decan, n-Undecan, n-Dodecan, n-Tridecan, n-Tetradecan, n-Pentadecan sowie die entsprechenden Alkene, insbesondere n-Pentadecen.

Der wässrigen Phase können neben der Lysesubstanz bzw. dem Chaotropsalz noch weitere Substanzen zugesetzt werden, wie beispielsweise zumindest eine Puffersubstanz und/oder zumindest ein Alkohol mit 1 bis 4 Kohlenstoffatomen und/oder zumindest ein Reduktionsmittel und/oder Puffer und/oder Reagenzien zur Aufhebung von durch Formalinbehandlung verursachten kovalenten Verknüpfungen/Modifikationen der Biomoleküle, wie Ammoniumchlorid und/oder Alkylamine.

Geeignete Puffersubstanzen sind beispielsweise Tris/HCl, Phosphat-Puffer, Borat-Puffer, PBS-Puffer, Citrat-Puffer, MES-Puffer oder HEPES-Puffer. Alkohole mit 1 bis 4 Kohlenstoffatomen sind beispielsweise Methanol, Ethanol, n-/iso-Propanol, Butanole bzw. Mischungen dieser Alkohole. Als Reduktionsmittel können DTT, Beta-Mercaptoethanol, TCEF oder andere Reduktionsmittel eingesetzt werden.

In weiterer Ausgestaltung des erfindungsgemäßen Verfahrens wird das Lösen des Paraffins unter Erwärmung durchgeführt, insbesondere unter Erwärmung auf eine Temperatur von 35 bis 70°C, bevorzugt auf 40 bis 60°C. Durch die Erwärmung wird ein schnelleres Lösen des Paraffins in dem nicht mit Wasser mischbaren Lösungsmittel begünstigt, wobei durch die moderaten Temperaturen eine thermische Veränderung des Probenmaterials weitgehend ausgeschlossen werden kann. Außerdem trägt diese Erwärmung bei gleichzeitigem Einsatz einer Lysesubstanz dazu bei, die Vernetzungen in Formalin-fixierten biologischen Proben aufzulösen.

In Weiterbildung des erfindungsgemäßen Verfahrens wird der Verfahrensschritt b) unter Erwärmung durchgeführt, insbesondere auf eine Temperatur von 70 bis 100°C. Die Erwärmung in diesem Schritt kann sowohl vor, während als auch nach Zugabe der wässrigen Lysesubstanz-Lösung bzw. des Wassers und der Lysesubstanz vorgenommen werden. Durch die Erwärmung kann die Lyse der Probe beschleunigt werden bzw. in vollständigerem Umfang ablaufen, wodurch die zu isolierenden Biomoleküle schneller bzw. möglichst quantitativ aus der biologischen Probe freigesetzt werden.

Bei der Wahl des nicht mit Wasser mischbaren Lösungsmittels ist es von Vorteil, wenn dieses einen Siedepunkt unter Normaldruck von wenigstens 150°C, insbesondere von wenigstens 160°C aufweist. Hierdurch wird verhindert, dass das zugesetzte organische Lösungsmittel während des Prozesses, insbesondere bei Erwärmung, zu stark verdampft und das Paraffin wieder ungelöst zurück lässt. Außerdem kann durch Verdampfen des organischen Lösungsmittels eine Geruchsbelästigung oder gar eine gesundheitliche Beeinträchtigung ausgehen. Ferner sind Lösungsmitteldämpfe häufig auch leicht entflammbar. Durch die Wahl eines Lösungsmittels mit verhältnismäßig hohem Siedepunkt wird dessen gasförmige Freisetzung bei einer gegebenen Temperatur reduziert, was die Brandgefahr verringert. Der geringe Dampfdruck solcher Lösungsmittel erlaubt auch ein zwischenzeitliches Verschließen des Probenbehälters, ohne dass dieser beim Erwärmen zu platzen droht.

Zur weiteren Verbesserung der Handhabungssicherheit werden nicht mit Wasser mischbare Lösungsmittel eingesetzt, die einen Flammpunkt von wenigstens 40°C, insbesondere von wenigstens 50°C, bevorzugt von wenigstens 60°C, weiter bevorzugt wenigstens 70°C oder gar wenigstens 80°C aufweisen. Dies sind beispielsweise Acetyl-Tributyl-Citrat, Adipinsäurediethylester, Decanol, Malonsäure-diethylester, Benzylalkohol oder Mischungen von diesen. Bevorzugt seien außerdem n-Dodecan, n-Tetradecan, n-Pentadecan, n-Pentadecen, Biodiesel, Sinarol oder Mischungen von diesen, die sich zusätzlich noch durch ein besonders gutes Phasentrennungsverhalten zur wässrigen Phase auszeichnen.

Die Isolierung der Biomoleküle aus der wässrigen Phase kann beispielsweise über eine Anlagerung der Biomoleküle an eine feste Phase vorgenommen werden, gewünschtenfalls gefolgt von einem oder mehreren Waschschritten. Als feste Phase kommen beispielsweise organische Polymerpartikel in Frage und/oder mineralische Trägermaterialien wie Quarzfasern, Silicagel, Glas, Aluminiumoxid, Zeolithe, Titandioxid und/oder Zirkondioxid. Diese Träger können in partikulärer Form vorliegen, vor allem als magnetische und/oder magnetisierbare Partikel, insbesondere Magnetbeads. Weiterhin kann der Träger als Fasern, Schwämme, Schäume, insbesondere in Form von Fritten oder Membranen eingesetzt werden. In bevorzugter Weise ist die feste Phase als chromatographische Säule ausgestaltet. Dabei kann die feste Phase die Säulenpackung und/oder eine Membran darstellen, wozu insbesondere die oben genannten organischen Polymerpartikel und/oder mineralische Trägermaterialien verwendet werden.

Als Waschlösung für die zuvor erwähnten Waschschritte können beispielsweise Hochsalzpuffer und/oder Hochalkohol verwendet werden. Unter Hochalkohol werden Alkohol/Wassermischungen mit einem hohen Alkoholgehalt von beispielsweise 70 oder 80 Vol.-% verstanden. Als Alkohole kommen beispielsweise Methanol, Ethanol, n-/iso-Propanol, Butanole bzw. Mischungen dieser Alkohole in Betracht. Die Hochalkohole können außerdem geringe Mengen an Puffersubstanzen enthalten.

Die Anlagerung der Biomoleküle an die feste Phase kann dadurch erzielt werden, dass die wässrige Phase vollständig und die organische Phase zumindest teilweise mit der festen Phase in Kontakt gebracht wird. Werden Silicamaterialien als feste Phase eingesetzt, kann die wässrige Phase zur Verbesserung der Bindebedingungen zuvor noch mit einem Chaotropsalz und/oder einem Alkohol mit einem bis vier Kohlenstoffatomen versetzt werden, wie beispielsweise Methanol oder Ethanol. Unter Verwendung eines organischen Lösungsmittels mit einer Dichte, die geringer als die von Wasser ist, kann dabei so vorgegangen werden, dass die sich unterhalb der organischen Phase befindliche wässrige Phase in einem bodenseitig spitz zulaufenden Probengefäß über eine unmittelbar über den Boden geführte Kanüle oder Pipette abgezogen wird, bis auch ein Teil der überstehenden organischen Phase mit überführt wird. Damit kann eine möglichst vollständige Überführung der Biomoleküle aus dem Probenbehälter erreicht werden. Das auf diese Weise abgesaugte Volumen kann anschließend auf eine chromatographische Säule gegeben werden, um die Biomoleküle an den festen Träger zu binden. Kontaminationen, die ebenfalls unter diesen Bedingungen an den festen Träger binden, können gewünschtenfalls über einen oder mehrere Waschschritte vollständig entfernt werden. Hierzu können Hochsalzpuffer und/oder Hochalkohol oder auch andere dem Fachmann für diese Zwecke bekannte Lösungen eingesetzt werden.

Bei Einsatz magnetischer oder magnetisierbarer Partikel, insbesondere Magnetbeads, können diese auch direkt zum zweiphasigen System aus organischer und wässriger Phase gegeben werden und die wässrige Phase mit den Partikeln anschließend abgetrennt werden. Sollte die organische Phase eine größere Dichte als die wässrige Phase aufweisen, werden die Partikel in diesem Schritt mit der organischen Phase abgetrennt. Mit dem Zusatz der Partikel kann zur Verbesserung der Bindebedingungen die wässrige Phase zuvor noch mit einem Chaotropsalz und/oder einem Alkohol mit einem bis vier Kohlenstoffatomen versetzt werden und/oder die Abtrennung der beladenen Partikel durch Zentrifugieren unterstützt werden.

Nach einer weiteren Ausführungsform des erfindungsgemäßen Verfahrens ist die wässrige und/oder organische Phase weitestgehend frei von Tensiden, insbesondere von anionischen, kationischen und/oder amphoteren Tensiden. Auf diese Weise kann eine deutliche Phasentrennung zwischen wässriger und organischer Phase sicher gestellt werden.

Ein weiterer Gegenstand der vorliegenden Anmeldung betrifft ein Kit zur Isolierung von Biomolekülen aus einer fixierten, in Paraffin eingebetteten biologischen Probe, insbesondere einer Gewebeprobe, das zumindest die folgenden Bestandteile enthält:
a) ein nicht mit Wasser mischbares Lösungsmittel und
b) mindestens eine Lysesubstanz und/oder eine wässrige Lösung mindestens einer Lysesubstanz.

In diesem Kit befinden sich die genannten Bestandteile bevorzugt in verschiedenen Behältnissen. Wenn die Lysesubstanz bereits als wässrige Lösung vorliegt, kann bei der Verwendung des Kits auf die weitere Zugabe von Wasser verzichtet werden.

In vorteilhafter Ausgestaltung des Kits enthält dieses eine Verfahrensanleitung, in der die Durchführung des erfindungsgemäßen Verfahrens abgedruckt ist. Dies gewährleistet eine möglichst fehlerfreie Benutzung des erfindungsgemäßen Kits.

Die vorliegende Anmeldung betrifft außerdem die Verwendung eines erfindungsgemäßen Kits zur Isolierung von Biomolekülen aus einer fixierten, in Paraffin eingebetteten biologischen Probe.

Im Folgenden wird das erfindungsgemäße Verfahren anhand von Ausführungsbeispielen genauer beschrieben.

### Beispiel 1: Isolierung von RNA aus Rattenleber FFPE-Proben

Zum Vergleich des erfindungsgemäßen Verfahrens mit dem aus dem Stand der Technik bekannten Verfahren unter Einsatz von Xylol zur Paraffinentfernung werden Probenschnitte mit gleicher Stärke von etwa 7,5 µm von Rattenleber FFPE-Proben den entsprechenden Verfahrensprotokollen unterzogen. Als feste Phase für die Isolierung der Nukleinsäuren werden kommerziell verfügbare Bindesäulen mit Silicamembran verwendet (NucleoSpin RNAXS, MACHEREY-NAGEL, Düren). Diese Säulen können in geeignete Collecting Tubes eingesteckt und leicht in jeder handelsüblichen Zentrifuge prozessiert werden.

| **Protokoll : Endparaffinisierung mit Xylol (Stand der Technik)** |
|---|
| 1 - 3 FFPE Schnitte von 7,5 µm Dicke je Präparation |
| Zugabe von 1 ml Xylo |
| Inkubation 2 min bei Raumtemperatur, 25°C (RT), 10 sec. vortexen |
| Zentrifugation max. Geschwindigkeit 2 min (ca. 14.000 x g) |
| Überstand und verwerfen |
| Zugabe von 1 ml Ethanol (~98%) |
| Vortexen für 2 sec. |
| Zentrifugation max. Geschwindigkeit 2 min |
| Überstand abpippetieren und verwerfen |
| Trocknen bei 60°C für 3-10 min mit offenem Deckel (oder länger bis Ethanol vollständig verdampft ist) |
| Zugabe von 100 µl Lysepuffer (3M GITC, 10 mM Tris, pH 7,0) |
| Zugabe von 10 µl Proteinase K (20 mg/ml) |
| vortexen 10 sec. |
| kurz zentrifugieren |
| Sollten Probenreste an der Wandung des Gefäßes sitzen, werden diese vorsichtig in die Lösung zurückgespült. Auf- und Abpipettieren der Lösung zur Homogenisierung. |
| Proteinase K Verdau bei 60°C für 15 min. Gefäß aus dem Inkubator entnehmen und für 5 sec vortexen. Sind noch Gewebereste sichtbar, sollte die Inkubation für bis zu 3 h fortgeführt werden. |
| Zugabe von 100 µl 200 mM Ammoniumchlorid |
| Vortexen |
| Inkubation bei 90°C für 15 min. Abkühlen lassen auf RT für ca. 2 min. |
| Zugabe von 200 µl Ethanol (96 -100%) |
| mixen |
| Kurz zentrifugieren (ca. 1 sec 100xg) |
| Mischung 3x auf- und abpipettieren und die Probe (410 µl) auf die NucleoSpin RNA XS Bindesäule geben und mit Standardprotokoll (siehe unten) fortfahren. |

| **Protokoll: Erfindungsgemäße Entparaffinisierung** |
|---|
| 1 - 3 FFPE Schnitte von 7,5 µm Dicke je Präparation |
| Zugabe von 0,3 ml Pentadecan |
| Inkubation für 2 min bei 60°C und sofort anschließen vortexen (um das Paraffin zu Schmelzen und zu lösen) |
| abkühlen lassen auf Raumtemperatur (5 min) |
| Zugabe von 100 µl Lysepuffer (3M GITC, 10 mM Tris, pH 7,0) |
| Vortexen, um das Gewebe aus der oberen organischen in die untere wässrige Phase zu transferieren |
| Zentrifugation für 1 min bei max. g-Zahl um die Phasentrennung und den Übergang des Gewebes in die wässrige Phase zu unterstützen. |
| Zugabe von 10 µl Proteinase K (20 mg/ml) zur wässrigen Phase. Mischen der wässrigen Phase durch auf- und abpipettieren. Nicht die wässrige mit der organischen Phase mischen. |
| Proteinase Verdau bei 60°C für 15 min. Gefäß aus dem Inkubator entnehmen und für 5 sec vortexen. Sind noch Gewebereste sichtbar, sollte die Inkubation für bis zu 3 h fortgeführt werden. |
| Zugabe von 100 µl 200 mM Ammoniumchlorid zur wässrigen Phase |
| Vortexen um das Ammoniumchlorid mit der wässrigen Phase zu mischen. Ggf. kurze Zentrifugation um die Phasentrennung zu unterstützen. |
| Inkubation bei 90°C für 15 min. Abkühlen lassen auf RT für ca. 2 min. |
| Zugabe von 200 µl Ethanol (96 -100%) |
| mixen |
| Kurz zentrifugieren (ca. 1 sec 1000xg) |
| Mischung 3x auf- und abpipettieren und die Proben (410 µl) auf die NucleoSpin RNA XS Bindesäule geben und mit RNA-Isolierungs-protokoll (siehe unten) fortfahren. Reste der organischen Phase die ggf. mit transferiert werden sind unkritisch. |

| **RNA-Isolierungsprotokoll nach Entparaffinisierung mit Xylol und erfindungsgemäßer Entparaffinisierung** | |
|---|---|
| 1 | Beladen der Spinsäulen und Zentrifugation bei 8.000xg für 30 s |
| 2 | Verwerfen des Colleting Tubes mit dem Durchlauf |
| 3 | Spinsäule in neues Collecting Tube stellen (2.0 ml) |
| 4 | Zugabe von 100 µl 75% Ethanol |
| 5 | Zentrifugation bei 11.000xg für 30 s |
| 6 | Zugabe von 25 µl DNase |
| 7 | bei 20 - 25°C für 15 min |
| 8 | Zugabe von 100 µl 2 M GITC, 10 mM Tris, pH 7,0, 75% Ethanol |
| 9 | Inkubation bei 20 - 25°C für 2 min |
| 10 | Zentrifugation bei 11.00xg für 30 s |
| 11 | Zugabe von 400 µl 75% Ethanol |
| 12 | Zentrifugation bei 11.000xg für 30 s |
| 13 | Verwerfen des Collecting Tubes mit dem Durchlauf |
| 14 | Spinsäule in neues Collecting Tube einsetzen (2.0 ml) |
| 15 | Zugabe von 200 µl 75% Ethanol |
| 16 | Zentrifugation bei 1.000xg für 2 min |
| 17 | Verwerfen des Collecting Tubes mit dem Durchlauf |
| 18 | Spinsäule in neues Collecting Tube einsetzen (1.5 ml) |
| 19 | Zugabe von 10 µl RNase-freiem Wasser |
| 20 | Zentrifugation bei 11.000xg für 30 s. Auffangen des Eluates im Collecting Tube und Lagerung der extrahierten RNA bei -20 oder -70°C. |

Die isolierte RNA wurde mittels qRT-PCR wie folgt quantifiziert:

| | |
|---|---|
| Target: | HPRT (Hypoxanthine guanine phosphoribosyl transferase) |
| Amplikongröße: | 101 bp |
| Spezifität: | Ratte, mRNA bzw. cDNA |
| PCR-Gerät: | LightCycler |
| Kit: | Sigma SYBR Green Quantitative RT-PCR Kit (#QR0100) |
| Templatemenge: | 2 µl |

Je Reaktionsansatz werden folgende Komponenten gemischt:

| | |
|---|---|
| Wasser | 3 µl |
| SYBR Green Taq Ready Mix for QRT-PCR (aus dem Kit: Sigma SYBR Green Quantitative RT-PCR Kit (#QR0100)) | 10 µl |
| 1:20 verdünnte DuraScript RT (aus oben genanntem Kit) | 1 µl |
| Primer A (10 µM = 10 pmol/µl) 5'-GCAGACTTTGCTTTCCTTGGT-3' | 2 µl |
| Primer B (10 µM = 10 pmol/µl) 5'-CTGGCCTGTATCCAACACTTC-3' | 2 µl |
| RNA-Eluat | 2 µl |
| Gesamtvolumen | 20 µl |

Der Reaktionsansatz wird in eine LightCycler Kapillare (Firma Roche) überführt. Mit folgendem Programm wird im LightCycler Instrument (Firma Roche) die Reaktion und Analyse durchgeführt.:

| | Target Temp (°C) | Incubation Time (sec) | Temperature Transition Rate (°C/sec) |
|---|---|---|---|
| Denaturierung | 48 | 1800 | 20 |
| Denaturierung | 94 | 30 | 20 |
| Amplifikation (40 Zyklen) | 94 | 5 | 20 |
| | 58 | 1 | 20 |
| | 72 | 2 | 20 |
| | 81 | 3 | 20 |
| Schmelzkurve | 95 | 0 | 20 |
| | 65 | 10 | 20 |
| | 95 | 0 | 0,1 |
| Cooling | 40 | 30 | 20 |

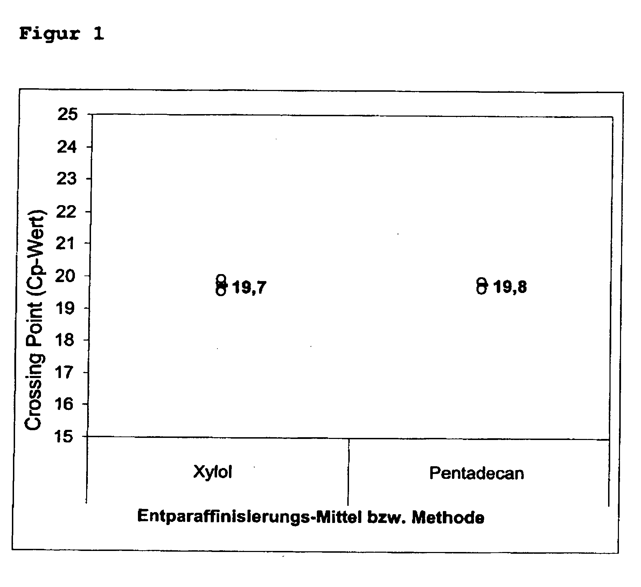

In der Figur 1 ist das Ergebnis der Messung dargestellt. Die erhaltenen Cp-Werte, die ein Maß für die erhaltene RNA-Menge darstellen, zeigen für beide Methoden gleiche Ergebnisse. Damit entspricht die RNA-Ausbeute für das erfindungsgemäße Verfahren weitestgehend der mittels der wesentlich aufwändigeren Xylol-Entparaffinisierung vorbehandelten Probe erhaltenen Menge.

### Beispiel 2: RNA und DNA-Isolierung aus Rattenleber FFPE-Proben

Bei diesem Versuch wird neben der RNA auch DNA aus den Proben isoliert, wobei die in Beispiel 1 beschriebenen Protokolle zur Entparaffinisierung mittels Xylol-Methode und dem erfindungsgemäßen Verfahren unverändert bleiben. Bezüglich der Nukleinsäureextraktion wird in Beispiel 2 auf den DNase-Verdau verzichtet und statt dessen eine selektive Elution der genomischen DNA vorgenommen.

| **Protokoll: DNA und RNA-Isolierung** | |
|---|---|
| Entparaffinisierung mit Xylol und nach dem erfindungsgemäßen Verfahren in Ausführungsbeispiel 1. | |
| Beladen der Bindesäule, Zentrifugation und Verwerfen des Collecting Tubes mit dem Durchfluss. | |
| Einstecken der Bindesäule mit der gebundenen RNA und DNA in eine neues Collecting Tube (2.0 ml). Abweichend vom Ausführungsbeispiel 1 wird zur Elution der DNA wie folgt verfahren: | |
| DNA steps | Zugabe von 100 µl 10 mM CaCl₂, 80% Ethanol, 10 mM Tris, pH 7,0 auf die Bindesäule |
| | Zentrifugation bei 11.000 x g für 30 s |
| | Es ist bei diesem Schritt nicht notwendig, das Collecting Tube nach der Zentrifugation auszutauschen. |
| | Erneute Zugabe von 100 µl 10 mM CaCl₂, 80% Ethanol, 10 mM Tris, pH 7,0 auf die Bindesäule |
| | für 2 min Zentrifugation bei 11.000 x g für 2 min |
| | Verwerfen des Collecting Tubes mit dem Durchlauf |
| | Einsetzen der Bindesäule in ein neues Collecting Tube (1.5 ml) |
| | Zugabe von 10 µl 10 mM CaCl₂, 10 Tris, pH 7,0 direkt auf das Zentrum der Silicamembran der Bindesäule |
| | Zentrifugation bei 11.000 x g für 30 s |
| | Lagerung der eluierten DNA für die spätere Analyse. |
| | Einsetzen der Bindesäule in ein neues Collectin Tube (2.0 ml) |
| Ab hier wird mit Schritt 6 des RNA-Isolierungsprotokolls gemäß Beispiel 1 fortgefahren. | |

Die Analyse der isolierten DNA erfolgt mittels qPCR, die wie folgt durchgeführt wurde:

| | |
|---|---|
| Target: | GAPDH |
| Amplikongröße: | 191 bp |
| Spezifität: | Maus, gDNA |
| PCR-Gerät: | LightCycler |
| Kit: | DyNamo Cypillary SYBR Green Kit (Finnzymes #F-420S/L) |
| Template-Menge: | 2 µl |

Zur Analyse der DNA-Eluate wird eine qPCR wie folgt durchgeführt.

Je Reaktionsansatz werden folgnde Komponenten gemischt:

| | |
|---|---|
| 2x DyNAmo master mix (aus Kit: DyNAmo Capillary SYBR Green qPCR Kit #F-420S/L) | 10 µl |
| Primer A (10 µM = 10 pmol/µl) 5'-AACGACCCCTTCATTGAC-3' | 0,5 µl |
| Primer B (10 µM = 10 pmol/µl) 5'-TCCACGACATACTCAGCAC-3' | 0,5 µl |
| Wasser | 7 µl |
| DNA-Eluat | 2 µl |
| Gesamtvolumen | 20 µl |

Der Reaktionsansatz wird in eine LightCycler Kapillare (Firma Roche) überführt. Mit folgendem Programm wird im LightCycler Instrument (Firma Roche) die Reaktion und Analyse durchgeführt.

| | Target Temp (°C) | Incubation Time (sec) | Temperature Transition Rate (°C/sec) |
|---|---|---|---|
| Denaturierung | 95 | 600 | 20 |
| Amplifikation (40 Zyklen) | 95 | 5 | 20 |
| | 55 | 10 | 20 |
| | 72 | 12 | 20 |
| Schmelzkurve | 95 | 10 | 20 |
| | 65 | 20 | 20 |
| | 95 | 0 | 0,2 |
| Cooling | 40 | 30 | 20 |

Die Analyse der isolierten RNA erfolgt mittels qRT-PCR wie in Beispiel 1 beschrieben.

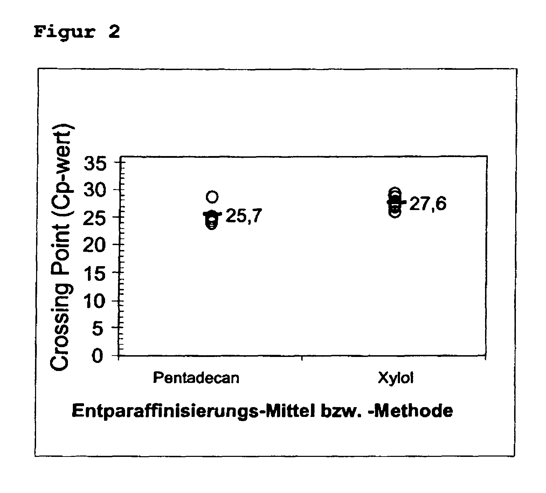

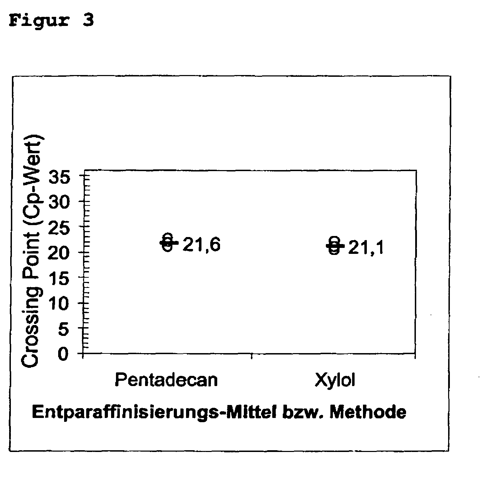

In Figur 2 sind die Ergebnisse der DNA-Analyse und in Figur 3 die Ergebnisse der RNA-Analyse dargestellt. Diese verdeutlichen, dass sich mit Hilfe des erfindungsgemäßen Isolierungsverfahrens neben der RNA auch DNA aus FFPE-Proben in Mengen isolieren lässt, die mit denen der Xylol-Entparaffinisierungsmethode vergleichbar sind. Das erfindungsgemäße Verfahren erreicht somit die Leistungsfähigkeit der Probenaufbereitung mittels Xylol-Methode unter Umgehung der mit dieser Methode verbundenen Nachteile.

### Beispiel 3: RNA-Isolierung aus Rattenleber FFPE-Proben mittels Biodiesel bzw. Sinarol als nicht mit Wasser mischbare Lösungsmittel

Die Behandlung der FFPE-Proben erfolgt wie im Ausführungsbeispiel 1 beschrieben, wobei die Entparaffinisierung gemäß des erfindungsgemäßen Verfahrens mit zwei unterschiedlichen Lösungsmitteln, nämlich Biodiesel bzw. Sinarol durchgeführt wurde. Bei Biodiesel (CAS-Nr. 67762-38-3) handelt es sich um eine Mischung verschiedener Fettsäuremethylester. Die Analyse der isolierten RNA erfolgte mittels qRT-PCR wie in Beispiel 1 beschrieben.

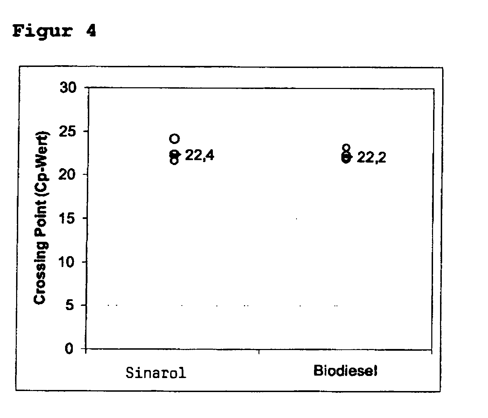

In Figur 4 ist das Ergebnis des Vergleichs dieser beiden Lösungsmittel dargestellt, die in Bezug auf die Ausbeute an isolierter RNA weitestgehend gleiche Ergebnisse liefern.

### Beispiel 4: RNA-Isolierung aus Rattenleber FFPE-Proben mit Pentadecan und dünnflüssigem Paraffin im Vergleich

In diesem Versuch wurde die Entparaffinisierung mittels Pentadecan und dünnflüssigem Paraffin (CAS-Nr. 8012-95-1) nach dem erfindungsgemäßen Verfahren gegenübergestellt. Hierzu wurden Rattenleber FFPE-Proben wie in Beispiel 1 beschrieben mit Pentadecan bzw. dünnflüssigem Paraffin als nicht mit Wasser mischbarem Lösungsmittel behandelt und die RNA aus den Proben isoliert.

Im Ergebnis zeigt sich, dass mit beiden Lösungsmitteln die Probe entparaffinisiert und die RNA anschließend isoliert werden kann. Pentadecan eignet sich im Vergleich zu dünnflüssigem Paraffin jedoch besser, da sich bei Pentadecan nach Zugabe des Lysepuffers und dem proteolytischen Aufschluss der Probe eine deutlichere und schärfere Phasengrenze zwischen der organischen und der wässrigen Phase ausbildet als bei der Verwendung von dünnflüssigem Paraffin. Letzteres neigt zur Bildung einer trüben Zwischenphase, in der sich möglicherweise Teile der Probe verfangen.

### Beispiel 5: Einfluss des K_{OW}-Wertes des nicht mit Wasser mischbaren Lösungsmittels auf die Paraffinentfernung

In den folgenden Versuchen wird das Lösen von Paraffin in verschiedenen erfindungsgemäß einsetzbaren nicht mit Wasser mischbaren Lösungsmitteln untersucht. Hierzu werden 82 mg Paraffin in 1640 µl Chloroform gelöst, so dass auf 1 mg Paraffin 20 µl Chloroform kommen. Hiervon werden aliquote Teile von 200 µl, 100 µl, 50 µl sowie 25 µl genommen, die 10, 5, 2,5 und 1,25 mg Paraffin entsprechen. Diese Proben werden in Eppendorf-Probengefäße eingefüllt und das Chloroform bei 60°C verdampft. Nachfolgend werden jeweils 1 ml der unten angegebenen Lösungsmittel hinzugegeben, das Paraffin unter Erwärmung auf 60°C gelöst, anschließend auf 20°C abgekühlt und die Löslichkeit des Paraffins im Lösungsmittel beurteilt. Die Ergebnisse sind in der folgenden Tabelle dargestellt.

| | Löslichkeit von Paraffin [mg] in 1 mL Lösemittel bei 20°C | | | | | | | |
|---|---|---|---|---|---|---|---|---|
| | Nonan | Xylol | Limonen | Chloroform | Pentadecan | Sinarol | Biodiesel | Propionsäurepentylester |
| mg/ml | 50 | 50 | 50 | 50 | Ca.40 | 33 | 25 | 10 |
| Kow-Wert | 5,4 | 3,1 | 3,4 | 2 | 7,7 | Ca.7 | Ca.8 | 2,9 |

| | | | | | | | |
|---|---|---|---|---|---|---|---|
| | | | | | | | |

| | Essigsäurebutylester | Decanol | Octansäure | Acetyltributylcitrat | Adipinsäurediethylester | Malonsäurediethylester | Benzylalkohol |
|---|---|---|---|---|---|---|---|
| mg/ml | 5 | 5 | 5 | 2,5 | 1,25 | <1,25 | <1,25 |
| Kow-Wert | 1,8 | 4,6 | 3,1 | 3,3 | 1,3 | 1 | 1,1 |

Die angegebenen K_{OW}-Werte sind den folgenden Quellen entnommen oder aus eigenen Experimenten abgeschätzt:
- BGIA - Institut für Arbeitsschutz der Deutschen Gesetzlichen Unfallversicherung GESTIS-Stoffdatenbank,
- LOGKOW - A database of evaluated octanol-water partition coefficients (Log P)
- PubChem Substance

Diese Ergebnisse zeigen, dass die Löslichkeit von Paraffinen in dem entsprechenden nicht mit Wasser mischbaren Lösemittel im Allgemeinen um so größer ist, je größer dessen Kow-Wert ist.

### Beispiel 6: Einfluss des K_{OW}-Wertes auf die Phasentrennung

In einem weiteren Versuch wurde die Phasentrennung zwischen wässriger und organischer Phase in Bezug auf die oben genannten Lösungsmittel untersucht. Hierzu wurden jeweils 400 µl des jeweiligen nicht mit Wasser mischbaren organischen Lösungsmittels mit 1,25 mg/ml Paraffin und 100 µl Lysepuffer gemischt, gefolgt von der Zugabe von 100 µl 200 mM Ammoniumchlorid. Anschließend werden 200 µl Ethanol (ungefähr 98 %ig) zugegeben und nach Mischen zur Unterstützung der Phasentrennung zentrifugiert. Auf diese Weise werden im Vergleich zum erfindungsgemäßen Verfahren vergleichbare Bedingungen geschaffen.

Zur Auswertung der Phasentrennung wurde das Volumen der wässrigen Phase gemessen. Die Summe der Bestandteile liegt unter Vernachlässigung der Volumenkontraktion bei etwa 400 µl. Bei Mischung mit einem nicht mit Wasser mischbaren Lösungsmittel, das einen niedrigen K_{OW}-Wert von beispielsweise 1,0 aufweist, ergibt sich jedoch ein deutlich größeres Volumen der wässrigen Phase von nahezu 600 µl. Hieraus folgt, dass Lösungsmittel mit einem niedrigen K_{OW}-Wert nach Ethanolzugabe eine schlechtere Trennung der wässrigen Phase von der organischen Phase ermöglichen. Lösungsmittel mit einem K_{OW}-Wert von 4,0 oder mehr liefern hingegen eine deutlich bessere Phasentrennung unter den oben genannten Bedingungen.

## Patentansprüche

1. Verfahren zur Isolierung von Biomolekülen aus einer fixierten, in Paraffin eingebetteten biologischen Probe, umfassend die Schritte:
Inkontaktbringen der Probe
a) mit einem nicht mit Wasser mischbaren Lösungsmittel und Lösen des Paraffins in diesem Lösungsmittel unter Ausbildung einer flüssigen organischen Phase, und
b) mit einer wässrigen Lösung mindestens einer Lysesubstanz und/oder mit Wasser und mindestens einer Lysesubstanz unter Ausbildung einer wässrigen Phase,
wobei durch die Schritte a) und b) ein zweiphasiges Gemisch gebildet wird; anschließend
Isolierung der Biomoleküle aus der wässrigen Phase.

2. Verfahren nach Anspruch 1, **dadurch gekennzeichnet, dass** der Schritt a) vor, nach oder gleichzeitig mit dem Schritt b) durchgeführt wird.

3. Verfahren nach Anspruch 1 oder 2 **dadurch gekennzeichnet, dass** das nicht mit Wasser mischbare Lösungsmittel eine geringere Dichte aufweist als Wasser und/oder das nicht mit Wasser mischbare Lösungsmittel bei ≤ 25 °C flüssig ist, insbesondere bei ≤ 20°C.

4. Verfahren nach einem der vorstehenden Ansprüche, **dadurch gekennzeichnet, dass** das nicht mit Wasser mischbare Lösungsmittel ausgewählt ist aus aromatischen Kohlenwasserstoffverbindungen mit 6 bis 30 Kohlenstoffatomen, aliphatischen Kohlenwasserstoffverbindungen mit 10 bis 30 Kohlenstoffatomen, Alkan- oder Alkensäuren mit 2 bis 20 und deren Derivaten, aromatischen oder aliphatischen Alkoholen mit 6 bis 12 Kohlenstoffatomen sowie aus Mischungen von diesen.

5. Verfahren nach einem der vorstehenden Ansprüche, **dadurch gekennzeichnet, dass** das nicht mit Wasser mischbare Lösungsmittel einen Siedepunkt unter Normaldruck von wenigstens 150 °C, insbesondere von wenigstens 160°C aufweist und/oder das nicht mit Wasser mischbare Lösungsmittel einen Flammpunkt von wenigstens 40°C, insbesondere von wenigstens 50°C aufweist und/oder einen K_{OW}-Wert von wenigstens 4 aufweist, insbesondere von wenigstens 5.

6. Verfahren nach einem der vorstehenden Ansprüche, **dadurch gekennzeichnet, dass** die Biomoleküle ausgewählt sind aus Proteinen, Nukleinsäuren, insbesondere aus einzel- oder doppelsträngigen Nukleinsäuren.

7. Verfahren nach einem der vorstehenden Ansprüche, **dadurch gekennzeichnet, dass** die Lysesubstanz ausgewählt ist aus Enzymen und/oder Chaotropsalzen.

8. Verfahren nach einem der vorstehenden Ansprüche, **dadurch gekennzeichnet, dass** im Schritt b) noch
• zumindest eine Puffersubstanz und/oder
• zumindest ein Alkohol mit einem bis vier Kohlenstoffatomen und/oder
• zumindest ein Reduktionsmittel und/oder
• zumindest ein Puffer und/oder Reagenz zur Aufhebung von durch Formalinbehandlung verursachten kovalenten Verknüpfungen und/ oder Modifikationen der Biomoleküle zugegeben wird.

9. Verfahren nach einem der vorstehenden Ansprüche, **dadurch gekennzeichnet, dass** das Lösen des Paraffins unter Erwärmung durchgeführt wird, insbesondere auf eine Temperatur von 35 bis 70°C und/oder der Verfahrensschritt b) unter Erwärmung durchgeführt wird, insbesondere auf eine Temperatur von 70 bis 100°C.

10. Verfahren nach einem der vorstehenden Ansprüche, **dadurch gekennzeichnet, dass** die Isolierung der Biomoleküle aus der wässrigen Phase über eine Anlagerung der Biomoleküle an eine feste Phase vorgenommen wird, die insbesondere eine chromatographische Säule ist, gewünschtenfalls gefolgt von einem oder mehreren Waschschritten, insbesondere zur Anlagerung der Biomoleküle an die feste Phase die wässrige Phase vollständig und die organische Phase zumindest teilweise mit der festen Phase in Kontakt gebracht wird.

11. Verfahren nach einem der vorstehenden Ansprüche, **dadurch gekennzeichnet, dass** die wässrige und/oder organische Phase weitestgehend frei von Tensiden, insbesondere von anionischen, kationischen und/oder amphoteren Tensiden ist.

## Claims

1. A method for isolating biomolecules from a fixed biological specimen embedded in paraffin, comprising the steps:
bringing the specimen into contact
a) with a solvent non-miscible with water and dissolving the paraffin in this solvent such as to form a liquid organic phase, and
b) with an aqueous solution of at least one lysis substance and/or with water and at least one lysis substance such as to form an aqueous phase;
whereas a two-phase mixture is formed by the steps a) and b); subsequent
isolation of the biomolecules from the aqueous phase.

2. The method according to Claim 1, **characterised in that** step a) is implemented before, after or at the same time as step b).

3. The method according to Claim 1 or 2 **characterised in that** the solvent non-miscible with water has a lesser density than water and/ or that the solvent non-miscible with water is liquid at ≤ 25 °C, in particular at ≤ 20°C.

4. The method according to any of the preceding claims, **characterised in that** the solvent non-miscible with water is selected from aromatic hydrocarbon compounds with 6 to 30 carbon atoms, aliphatic hydrocarbon compounds with 10 to 30 carbon atoms, alkanoic or alkenoic acids with 2 to 20 carbon atoms, and derivatives of the latter, aromatic or aliphatic alcohols with 6 to 12 carbon atoms and mixtures of the latter.

5. The method according to any of the preceding claims, **characterised in that** the solvent non-miscible with water has a boiling point under normal pressure of at least 150 °C, in particular at least 160°C and/ or that the solvent non-miscible with water has a flash point of at least 40°C, in particular of at least 50°C and/ or that the solvent non-miscible with water has a K_{OW} value of at least 4, in particular of at least 5.

6. The method according to any of the preceding claims, **characterised in that** the biomolecules are selected from proteins, nucleic acids, in particular from single- or double-stranded nucleic acids.

7. The method according to any of the preceding claims, **characterised in that** the lysis is selected from enzymes and/or chaotropic salts.

8. The method according to any of the preceding claims, **characterised in that** in step b)
• at least one buffer substance and/or
• at least one alcohol with one to four carbon atoms and/or
• at least one reducing agent and/or
• at least one buffer and/or reagent are also added in order to eliminate covalent combinations and/or modifications of the biomolecules caused by formalin treatment.

9. The method according to any of the preceding claims, **characterised in that** the dissolution of the paraffin is implemented by heating, in particular to a temperature of 35 to 70°C and/ or that procedural step b) is implemented by heating, in particular to a temperature of 70 to 100°C.

10. The method according to any of the preceding claims, **characterised in that** the isolation of the biomolecules from the aqueous phase is undertaken by adding the biomolecules to a solid phase, in particular a chromatographic column, if so desired followed by one or more washing steps, especially in order to add the biomolecules to the solid phase the aqueous phase is brought totally and the organic phase at least partially into contact with the solid phase.

11. The method according to any of the preceding claims, **characterised in that** the aqueous and/or organic phase is free as far as possible from tensides, in particular from anionic, cationic and/or amphoteric tensides.

## Revendications

1. Procédé pour l'isolation de biomolécules issues d'un échantillon biologique fixé enrobé dans de la paraffine, comprenant les étapes suivantes:
Mise en contact de l'échantillon
a) avec un solvant non miscible avec de l'eau et dissolution de la paraffine dans ce solvant avec formation d'une phase liquide organique, et
b) avec une solution aqueuse au moins une substance de lyse et / ou avec de l'eau et au moins une substance de lyse sous formation d'une phase aqueuse.
sachant que par la phase a) et b), un mélange à deux phases est formé ; puis
isolation de la biomolécule issue de la phase aqueuse.

2. Procédé selon revendication 1, **caractérisé en ce que** l'étape a) est réalisée avant, après ou même temps que l'étape b).

3. Procédé selon revendication 1 ou 2, **caractérisé en ce que** le solvant non miscible avec de l'eau présente une densité inférieure à l'eau et / ou le solvant non miscible avec de l'eau est liquide à ≤ 25°C, notamment à ≤ 20°C.

4. Procédé selon l'une des revendications précédentes, **caractérisé en ce que** le solvant non miscible avec de l'eau est sélectionné parmi des liaisons aromatiques hydrocarbures avec de 6 à 30 atomes de carbure d'hydrogène, des liaisons hydrocarbures aliphatiques avec de 10 à 30 atomes de carbure d'hydrogène, des acides d'alcane ou acides alcènes avec de 2 à 20 et leurs dérivés, des alcools aromatiques ou aliphatiques avec de 6 à 12 atomes de carbone et des mélanges de ceux-ci.

5. Procédé selon l'une des revendications précédentes, **caractérisé en ce que** le solvant non miscible avec de l'eau présente un point d'ébullition sous pression normale d'au moins 150°C, notamment d'au moins 160°C et / ou le solvant non miscible avec de l'eau présente un point de flamme d 'au moins 40°C, notamment d'au moins 50°C et / ou une valeur K_{ow} d'au moins 4, notamment au moins 5.

6. Procédé selon l'une des revendications précédentes, **caractérisé en ce que** les biomolécules sont sélectionnées parmi des protéines, des acides nucléiques, notamment des acides nucléiques à chaîne unique ou double.

7. Procédé selon l'une des revendications précédentes, **caractérisé en ce que** la substance de lyse est sélectionnée parmi des enzymes et / ou sels chaotropiques.

8. Procédé selon l'une des revendications précédentes, **caractérisé en ce que** dans l'étape b)
• au moins une substance tampon et /ou
• au moins un alcool avec de un à quatre atomes carbone et / ou
• au moins un agent de réduction et / ou
• au moins un tampon et / ou réactif pour la neutralisation de liaisons covalentes et / ou modifications de la biomolécule provoquées par le traitement à la formaline est ajouté.

9. Procédé selon l'une des revendications précédentes, **caractérisé en ce que** la dissolution de la paraffine est réalisée par réchauffement, notamment à une température de 35 à 70°C et / ou l'étape de procédé b) est réalisée avec réchauffement notamment à une température de 70 à 100°C.

10. Procédé selon l'une des revendications précédentes, **caractérisé en ce que** l'isolation de la biomolécule issue de la phase aqueuse est effectuée par une addition de la biomolécule à une phase consistante, qui notamment est une colonne chromatographique, et au cas où on le désire, suivi d'une ou plusieurs étapes de lavage, notamment pour l'addition de la biomolécule sur la phase consistante la phase aqueuse entièrement et la phase organique au moins en partie est mise en contact avec la phase consistante.

11. Procédé selon l'une des revendications précédentes, **caractérisé en ce que** la phase aqueuse et / ou organique est, dans une large mesure, libre d'agents tensioactifs, notamment d'agents tensioactifs anioniques, cationiques et / ou amphotères.
